# FASCICULE DE BREVET EUROPEEN

(11) **EP 3 077 500 B1**
(45) Date de publication et mention de la délivrance du brevet: **06.09.2017**
(21) Numéro de dépôt: 14806236.7
(22) Date de dépôt: 01.12.2014
(51) Int. Cl.: C12M 1/34, C12Q 1/02, C12M 3/06, C12M 1/00, C12M 1/26

(54) **INSTALLATION DE COUPLAGE D'UN BIORÉACTEUR AVEC UN APPAREIL D'ANALYSE PHYSICO-CHIMIQUE OU DE COLLECTE D'ÉCHANTILLONS**
ANLAGE ZUR KOPPLUNG EINES BIOREAKTORS MIT EINER VORRICHTUNG ZUR PHYSIKALISCH-CHEMISCHEN ANALYSE ODER PROBENAHME
FACILITY FOR COUPLING A BIOREACTOR WITH A DEVICE FOR PHYSICOCHEMICALLY ANALYSING OR COLLECTING SAMPLES

(30) Priorité: 06.12.2013 FR 1362258
(43) Date de publication de la demande: 12.10.2016
(73) Titulaire: Université Technologie de Compiègne-UTC, 60200 Compiègne (FR); Centre National de la Recherche Scientifique (C.N.R.S.), 75016 Paris (FR)
(72) Inventeur: LECLERC, Eric, F-60280 Margny Les Compiegne (FR); PAULLIER, Patrick, F-60150 Thourotte (FR); MERLIER, Franck, 60750 Choisy-Au-Bac (FR)
(74) Mandataire: Regimbeau
(86) Numéro de dépôt international: PCT/EP2014/076081
(87) Numéro de publication internationale: WO 2015/082392

(56) Documents cités:
- WO-A1-2005/019805
- FR-A1- 2 957 087
- US-A1- 2007 020 751
- PAUL M. VAN MIDWOUD ET AL: "On-line HPLC Analysis System for Metabolism and Inhibition Studies in Precision-Cut Liver Slices", ANALYTICAL CHEMISTRY, vol. 83, no. 1, 1 janvier 2011 (2011-01-01), pages 84-91, XP055144031, ISSN: 0003-2700, DOI: 10.1021/ac1018638
- GRIST SAMANTHA ET AL: "Microfluidic cell culture systems with integrated sensors for drug screening", MICROFLUIDICS, BIOMEMS, AND MEDICAL MICROSYSTEMS X, SPIE, 1000 20TH ST. BELLINGHAM WA 98225-6705 USA, vol. 8251, no. 1, 8 mars 2012 (2012-03-08) , pages 1-12, XP060023608, DOI: 10.1117/12.911427
- PAUL M. VAN MIDWOUD ET AL: "Microfluidic devices for in vitro studies on liver drug metabolism and toxicity", INTEGRATIVE BIOLOGY, vol. 3, no. 5, 1 janvier 2011 (2011-01-01) , page 509, XP055144861, ISSN: 1757-9694, DOI: 10.1039/c0ib00119h
- MIN-HSIEN WU ET AL: "Microfluidic cell culture systems for drug research", LAB ON A CHIP, vol. 10, no. 8, 1 janvier 2010 (2010-01-01), page 939, XP055145089, ISSN: 1473-0197, DOI: 10.1039/b921695b
- JEAN MATTHIEU PROT ET AL: "The Current Status of Alternatives to Animal Testing and Predictive Toxicology Methods Using Liver Microfluidic Biochips", ANNALS OF BIOMEDICAL ENGINEERING, KLUWER ACADEMIC PUBLISHERS-PLENUM PUBLISHERS, NE, vol. 40, no. 6, 10 décembre 2011 (2011-12-10), pages 1228-1243, XP035062589, ISSN: 1573-9686, DOI: 10.1007/S10439-011-0480-5

## Description

### DOMAINE TECHNIQUE GENERAL

L'invention se situe dans le domaine de l'analyse en continu et en temps réel, ou quasiment en continu et en temps réel, d'un fluide biologique issu d'au moins un bioréacteur de culture de bio-organismes, tels des cellules, des levures ou des bactéries, par exemple.

Elle concerne plus précisément une installation de couplage entre ce bioréacteur de culture en mode dynamique et au moins un appareil d'analyse physico-chimique, tel qu'un dispositif de chromatographie liquide ou un spectromètre de masse, par exemple ou un appareil de collecte d'échantillons.

### ETAT DE L'ART

Parmi les cultures de bio-organismes, les cultures cellulaires *in vitro* sont de plus en plus souvent utilisées dans toutes les phases de la recherche pharmaceutique, car elles représentent une alternative avantageuse aux modèles *in vivo,* c'est-à-dire à l'expérimentation animale, contre laquelle des pressions à la fois économiques et éthiques apparaissent au niveau international.

Ces cultures *in vitro* permettent de cultiver des cellules d'un tissu ou d'un organe animal ou humain, de façon à ce qu'elles miment le comportement biologique du tissu ou de l'organe pris dans son environnement naturel, au sein d'un organisme vivant.

Ces cultures cellulaires permettent ainsi de prévoir la réaction du tissu ou de l'organe naturel vis-à-vis des xénobiotiques, c'est-à-dire des molécules étrangères à un organisme vivant, par exemple des médicaments, des pesticides, des polluants industriels.

Plus précisément, ces cultures permettent de détecter et d'analyser les métabolites produits par les cellules, (c'est-à-dire les produits de transformation et/ou de dégradation du produit initial), de détecter des variations du "métabolome" (c'est à dire l'ensemble des métabolites et autres molécules que l'on peut trouver dans un fluide biologique) et de suivre et de rechercher des biomarqueurs. Ces cultures constituent ainsi un outil d'évaluation du métabolisme et de la toxicité des xénobiotiques.

Actuellement, les cultures cellulaires se font principalement sur des boites de Pétri. Ces boites contentant un milieu nutritif et placées dans un environnement favorable à la croissance cellulaire, sont ensemencées avec les cellules du tissu ou de l'organe à simuler. Une fois celles-ci développées, elles sont mises en contact avec le xénobiotique à analyser. L'impact sur les cellules est ensuite déterminé via le prélèvement et l'analyse des métabolites.

Ces boites de Pétri présentent des inconvénients.

Tout d'abord, elles ne permettent ni d'obtenir des cultures de cellules multicouches (tridimensionnelles), ni de maintenir en vie longtemps certaines catégories de cellules, notamment les hépatocytes primaires, par exemple. Or, il est justement important de pouvoir étudier les cellules de foie ou de rein, car ces organes interviennent majoritairement dans le métabolisme des xénobiotiques.

Par ailleurs, ce type de culture est inapte à reproduire le comportement réel d'un tissu ou d'un organe dans un organisme vivant.

De plus, les cellules sur boites de Pétri sont cultivées dans des conditions "statiques" (c'est-à-dire sans circulation de fluides) et non "dynamiques", (avec circulation de fluides), alors que ces dernières sont justement les seules susceptibles de refléter le comportement réel d'un tissu ou d'un organe irrigué.

Enfin, les cultures sur boites de Pétri ne permettent pas ou difficilement d'étudier les expositions chroniques à des xénobiotiques à faibles doses.

Pour ces raisons, il a été proposé de remplacer les boites de Pétri par des bioréacteurs fonctionnant en "mode dynamique".

La notion de "mode dynamique" signifie que le fluide dans lequel baigne la culture est mû en circulation par une pompe.

Parmi ces bioréacteurs, on trouve les "biopuces micro-fluidiques".

Par le terme "biopuce micro-fluidique", on désigne un dispositif miniaturisé qui comprend une chambre de culture "micro-structurée" (ou compartiment), c'est à dire dont au moins l'une des parois supérieure ou inférieure présente des microstructures, c'est-à-dire des canaux de quelques dizaines à quelques centaines de micromètres, fréquemment compris entre 20 µm et 500 µm.

Ces biopuces permettent ainsi une culture cellulaire en trois dimensions, dans un environnement dynamique et micro-fluidique et sont donc les mieux à mêmes de reproduire *in vitro* les conditions observées *in vivo.*

Les xénobiotiques sont introduits dans la boucle de circulation du fluide nutritif, en amont de la biopuce et les métabolites et marqueurs biologiques sont prélevés en aval de la biopuce.

Ces prélèvements sont ensuite analysés dans des appareils de mesure, tels que les appareils de chromatographie liquide ou gazeuse ou un spectromètre de masse.

S'il est ainsi possible d'effectuer des prélèvements à intervalles de temps successifs et de les analyser, cette technique ne permet pas de suivre en continu le métabolisme du xénobiotique étudié.

En outre, si certains des biomarqueurs et métabolites produits sont instables, présentent des durées de vie courtes, ou sont produits en faible quantité, il existe un risque qu'ils ne soient pas détectés entre deux prélèvements successifs.

Or, il pourrait être intéressant par exemple de suivre en temps réel des phénomènes de régénération cellulaire, de migrations cellulaires ou de clairance (capacité d'un organe à éliminer une substance donnée), de suivre des processus d'inflammations, des réponses transitoires ou encore des phénomènes de différentiation cellulaires. Avec les techniques précitées, ceci n'est pas possible ou du moins est très difficile et les résultats obtenus manquent de précisions temporelles.

On connait déjà d'après l'article de Paul van Midwoud et al., "On line HPLC analysis system for metabolism and inhibition studies in precision-cut liver slices", Anal. Chem. 2011, 83, 84-91, une installation de couplage d'une biopuce à trois chambres avec un appareil d'analyse par chromatographie liquide haute performance (HPLC). La biopuce est alimentée en amont en milieu de culture et/ou en inhibiteur à étudier. En outre, chacune des trois sorties de la biopuce est connectée à une boucle d'injection, via une vanne à six voies, elle-même montée sur le circuit de l'appareil HPLC entre la pompe et la colonne d'analyse.

Une telle installation de couplage permet d'injecter séquentiellement des échantillons issus de l'une des chambres de la biopuce dans l'appareil de chromatographie, une fois toutes les 30 minutes comme mentionné dans cet article.

Toutefois, dans cette installation, le circuit d'alimentation (perfusion) de la biopuce n'est pas fermé. Il s'ensuit qu'à intervalles réguliers, une partie des fluides issus de la biopuce est dirigée vers un réservoir de vidange et ne retourne pas enrichir la biopuce. Par ailleurs, la boucle d'injection peut s'encrasser à la longue et ne peut pas être nettoyée.

### PRESENTATION DE L'INVENTION

L'invention a pour but de résoudre les inconvénients précités de l'état de la technique en proposant une installation qui permette de coupler un bioréacteur de culture en mode dynamique à un ou plusieurs appareil(s) d'analyse(s) physico-chimique(s) ou de collecte d'échantillons, afin de réaliser une analyse en temps réel et continue du fluide issu de ce bioréacteur.

Un autre objectif de l'invention est de fournir une installation qui permette éventuellement de piéger un échantillon de fluide pendant une certaine période de temps, avant son injection dans l'appareil d'analyse, et ce, afin d'augmenter la concentration des métabolites ou biomarqueurs recueillis.

Un objectif additionnel de l'invention est de fournir une installation qui puisse être nettoyée facilement et fréquemment, sans pour autant contaminer les échantillons prélevés issus du bioréacteur.

L'invention a également pour objectif de fournir une installation qui permette d'éviter une perte de signal dans le temps, en cas d'encrassage ou de début d'encrassage de l'appareil d'analyse.

Enfin, l'installation de couplage proposée devrait avantageusement être facilement transportable, simple à fabriquer et à utiliser.

A cet effet, l'invention concerne une installation de couplage d'un bioréacteur de culture de bio-organismes, en mode dynamique, avec au moins un appareil d'analyse physico-chimique d'un fluide issu de ce bioréacteur ou de collecte d'échantillons de ce fluide, ledit appareil étant équipé d'une pompe et d'un injecteur, le bioréacteur étant équipé de moyens d'alimentation en milieu de culture et en xénobiotique(s).

Conformément à l'invention, le bioréacteur est raccordé à une boucle de perfusion fermée pourvue d'une pompe, cette boucle de perfusion étant munie d'une boucle de dérivation, dite "boucle de prélèvement" et en ce que l'installation comprend :
- un dispositif d'injection d'un produit de nettoyage dans ladite boucle de prélèvement,
- une première vanne comprenant six voies,
- et une deuxième vanne comprenant six voies,
la première vanne étant connectée à la boucle de perfusion fermée, à la boucle de prélèvement et à une boucle de nettoyage, la deuxième vanne étant raccordée à la boucle de nettoyage et à une boucle d'injection qui relie l'injecteur à au moins l'un des appareils d'analyse physico-chimique ou de collecte d'échantillons.

Selon d'autres caractéristiques avantageuses et non limitatives de l'invention, prises seules ou en combinaison :
- l'installation comprend une troisième vanne munie d'au moins une entrée et de deux sorties, cette troisième vanne étant positionnée sur la boucle d'injection, de façon que son entrée soit connectée à l'injecteur, que sa première sortie soit connectée à au moins l'un appareils d'analyse ou de collecte et que sa deuxième sortie soit connectée à un réservoir de vidange, ladite troisième vanne occupant soit une première position dans laquelle l'entrée est connectée à sa première sortie, soit une seconde position dans laquelle l'entrée est connectée à sa deuxième sortie ;
- pour la première vanne, ses premier et deuxième orifices de raccordement sont connectés à la boucle de perfusion fermée du bioréacteur, ses troisième et sixième orifices de raccordement sont connectés à la boucle de prélèvement, son quatrième orifice de raccordement au troisième orifice de raccordement de la deuxième vanne et son cinquième orifice de raccordement au sixième orifice de raccordement de la deuxième vanne, ladite première vanne occupant soit une première position, dans laquelle son premier orifice de raccordement est connecté à son deuxième, son troisième à son quatrième et son cinquième à son sixième, soit une seconde position, dans laquelle son premier orifice de raccordement est connecté à son sixième, son deuxième à son troisième et son quatrième à son cinquième,
- pour la deuxième vanne, son premier orifice de raccordement est connecté à un réservoir de vidange, son deuxième orifice de raccordement au dispositif d'injection d'un produit de nettoyage, son cinquième orifice de raccordement à l'injecteur et son quatrième orifice de raccordement à au moins l'un des appareils d'analyse physico-chimique, cette deuxième vanne occupant soit une première position dans laquelle son premier orifice de raccordement est connecté à son deuxième, son troisième à son quatrième et son cinquième à son sixième, soit une seconde position dans laquelle son premier orifice de raccordement est relié à son sixième, son deuxième à son troisième et son quatrième à son cinquième ;

- le quatrième orifice de raccordement de la deuxième vanne est connecté à l'entrée de la troisième vanne ;
- la boucle de prélèvement inclut une colonne de piégeage ;
- un filtre est présent sur la boucle de perfusion entre le bioréacteur et la première vanne ;
- le bioréacteur est placé à l'intérieur d'une enceinte dans laquelle le taux de dioxyde de carbone et la température sont contrôlés ;
- l'appareil d'analyse physico-chimique est un appareil de chromatographie liquide ;
- l'appareil d'analyse physico-chimique est un appareil de chromatographie liquide haute précision HPLC ;
- l'appareil d'analyse physico-chimique est choisi parmi un spectromètre de masse, notamment un spectromètre de masse doté d'une source d'ionisation pour liquide de type ESI, APCI ou APPI, un appareil de résonance magnétique nucléaire RMN, un détecteur fluorométrique, un spectrophotomètre ou un appareil de couplage en ligne MALDI TOF ;
- l'appareil est un collecteur de fractions permettant de collecter des échantillons du fluide issu du bioréacteur ;
- l'installation comprend un ou plusieurs appareil(s) d'analyse physico-chimique, monté(s) en série après un premier appareil d'analyse physico-chimique, ce premier appareil étant notamment un appareil de chromatographie liquide ;
- le bioréacteur est une boite multi-réacteurs ;
- le bioréacteur est une biopuce ;
- la biopuce est de type micro-fluidique.

L'invention concerne également un procédé d'analyse en ligne d'un fluide biologique issu d'un bioréacteur ou de collecte d'échantillons de ce fluide avec l'installation précitée, qui consiste à réaliser au moins une fois le cycle d'étapes suivant :
- a) mettre la première vanne dans sa première position et la deuxième vanne dans sa seconde position, de façon à nettoyer et sécher la boucle de prélèvement,
- b) mettre la première vanne et la deuxième vanne dans leur seconde position, de façon à recueillir un échantillon dans la boucle de prélèvement et à injecter un étalon interne dans l'appareil à partir de l'injecteur,
- c) mettre la première vanne et la deuxième vanne dans leur première position, de façon à injecter l'échantillon recueilli dans la boucle de prélèvement, dans l'appareil, à l'aide d'un éluant fourni par l'injecteur.

### PRESENTATION DES FIGURES

D'autres caractéristiques et avantages de l'invention apparaîtront de la description qui va maintenant en être faite, en référence aux dessins annexés, qui en représentent, à titre indicatif mais non limitatif, différents modes de réalisation possibles.

Sur ces dessins :

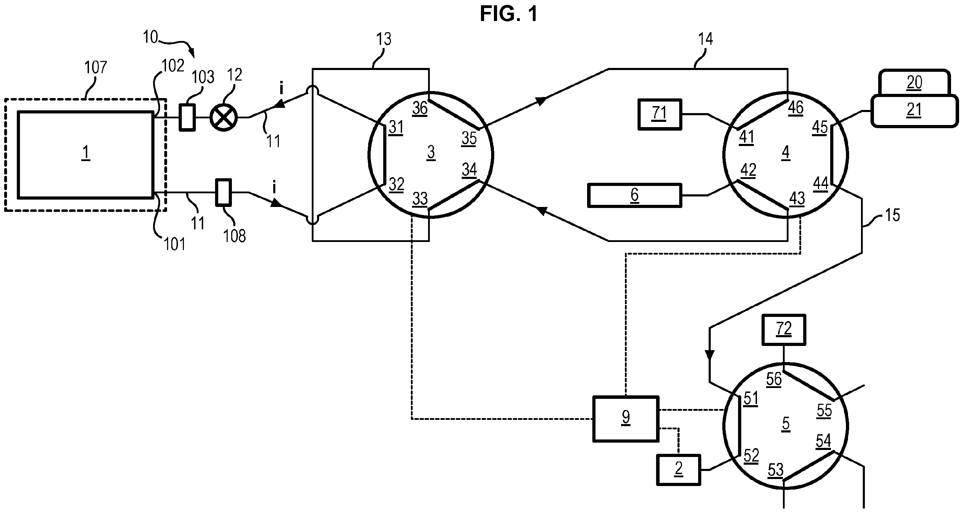

- les figures 1 à 3 sont des schémas représentant différents modes de réalisation de l'installation de couplage conforme à l'invention, dans trois états de fonctionnement différents.

### DESCRIPTION DETAILLEE

En se reportant aux figures précitées, on peut voir que l'installation conforme à l'invention permet de coupler un bioréacteur 1 avec au moins un appareil 2.

Le bioréacteur 1 est du type qui permet la culture de bio-organismes, en mode dynamique.

Les bio-organismes sont préférentiellement des cellules mais peuvent également être des bactéries ou des levures par exemple.

Le bioréacteur comprend un ou plusieurs compartiments à l'intérieur duquel ou desquels sont cultivés les bio-organismes.

Le bioréacteur 1 est muni d'une boucle de perfusion fermée 11 qui relie sa sortie 101 à son entrée 102. En outre, une pompe 12 est installée, par exemple sur la branche retour de cette boucle de perfusion fermée 11, de façon à maintenir en circulation le fluide biologique. Le sens de circulation est matérialisé par les flèches i.

La pompe 12 est de préférence une pompe péristaltique qui permet d'entrainer le fluide présente dans le tube souple constituant la boucle 11 par compression et déformation de celui-ci depuis l'extérieur. Une telle pompe est particulièrement adaptée aux petits débits et ne risque pas de contaminer le fluide du fait qu'elle ne comprend pas d'éléments en contact direct avec celui-ci.

Le bioréacteur 1 est en outre équipé de moyens 10 d'alimentation en milieu de culture.

Selon un premier mode de réalisation représenté sur la figure 1, ces moyens 10 sont constitués par un réservoir 103 raccordé à la boucle de perfusion 11, en aval de la pompe 12 et en amont de l'entrée 102 du bioréacteur 1. Le milieu de culture est placé dans ce réservoir et est renouvelé, tant que de besoin. Par ailleurs, le ou les xénobiotique(s) sont introduits dans ce réservoir 103 aux fins de leur analyse. La pompe 12 permet ainsi d'alimenter le bioréacteur 1 en milieu de culture, mais également de faire recirculer le fluide qui en sort.

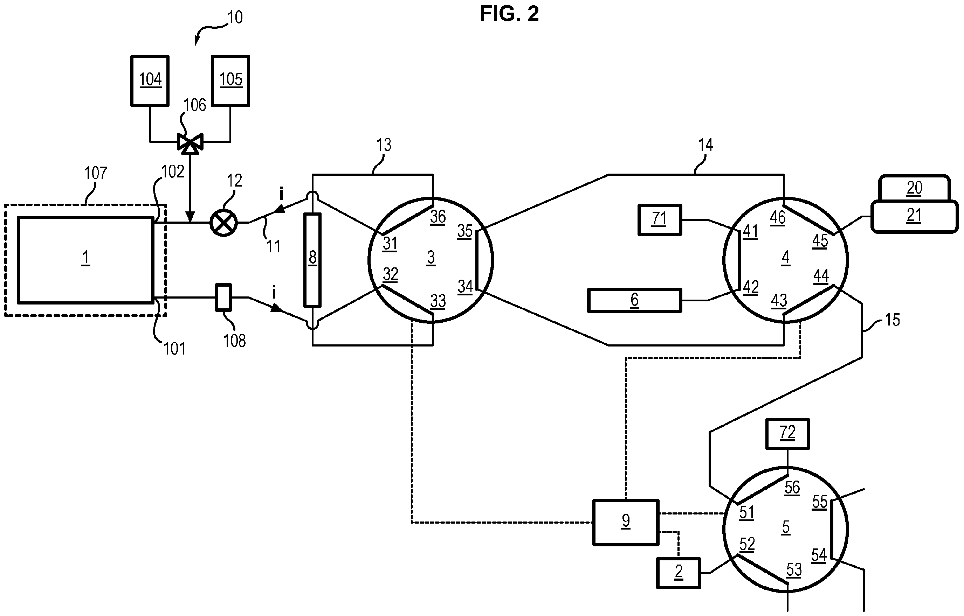

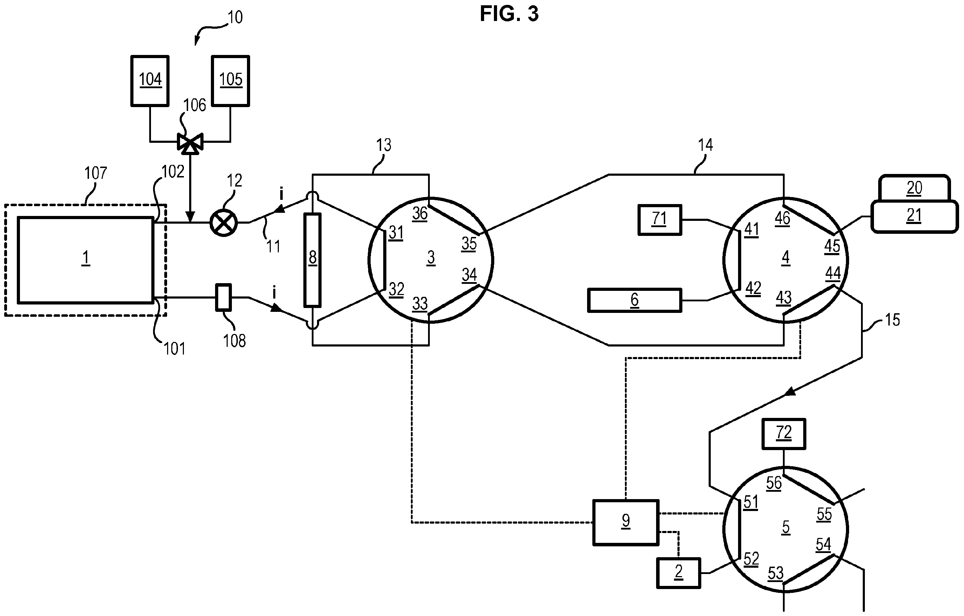

Selon un second mode de réalisation représenté sur les figures 2 et 3, les moyens 10 sont constitués d'un dispositif automatisé qui comprend un réservoir 104 de milieu de culture, un réservoir 105 de xénobiotiques, tous les deux raccordés à une vanne à trois voies 106 dont la sortie est à son tour raccordée en un point de la boucle fermée 11, situé entre la pompe 12 et l'entrée 102 du bioréacteur 1.

Le bioréacteur 1 peut par exemple être une boîte multi-réacteurs qui comprend plusieurs compartiments de cultures, de préférence micro-structurées, chacune étant connectée à un port d'entrée via un puits d'entrée et à un port de sortie via un puits de sortie. Un compartiment "micro-structuré" signifie que le volume de liquide qui y est stocké est de quelques microlitres. Des exemples de telles boîtes multi-réacteurs sont décrits par exemple dans les documents FR 2 957 086 et FR 2 957 087.

Le bioréacteur 1 peut également être constitué d'une biopuce micro-fluidique ou non, qui comprend dans ce cas un seul compartiment de culture.

De préférence, le bioréacteur 1 est disposé à l'intérieur d'une enceinte 107 à l'intérieur de laquelle le taux de dioxyde de carbone (CO₂), ainsi que la température sont contrôlés, de façon à maintenir des conditions de culture optimales des bio-organismes présents dans ce bioréacteur.

Enfin, on notera que de façon avantageuse, un filtre 108 peut être disposé sur la boucle de perfusion 11, entre le bioréacteur 11 et l'orifice de raccordement 32 de la vanne 3.

De façon avantageuse, le bioréacteur et son enceinte 107 sont de dimensions suffisamment petites pour être portables et notamment transportables à proximité des appareils 2, 2'.

L'appareil 2 est relié à un injecteur 21, via une boucle d'injection 15.

Cet injecteur 21 est lui-même connecté à une pompe 20, montée sur un réservoir non représenté sur les figures.

L'injecteur 21 permet d'envoyer dans l'appareil 2 au choix, soit un éluant, soit un étalon permettant de calibrer et d'étalonner les courbes obtenues par l'appareil 2, comme cela sera précisé ultérieurement.

Selon une première variante de réalisation, l'installation comprend un seul appareil 2.

De préférence, cet appareil 2 est un appareil de chromatographie en phase liquide, (par exemple du type HPLC), qui permet d'effectuer une analyse quantitative, qualitative et séparative du fluide issu du bioréacteur 1. Ce type de chromatographie repose sur la séparation des composés entraînés par l'éluant, à travers une phase stationnaire.

Toutefois, cet appareil 2 peut également être un appareil d'analyse physico-chimique choisi, par exemple, parmi :
un spectromètre de masse, notamment un spectromètre de masse doté d'une source d'ionisation pour liquide du type ionisation par électronébulisation ("ESI", de l'anglais "Electro Spray Ionisation"), du type ionisation chimique à pression atmosphérique ("APCI", de l'anglais "Atmospheric Pressure Chemical Ionisation") ou du type photoionisation à pression atmosphérique ("APPI", de l'anglais "Atmospheric Pressure Photo Ionisation"), un appareil d'analyse par résonnance magnétique nucléaire (RMN), un détecteur fluorométrique, un spectrophotomètre ou un appareil de couplage en ligne de type "MALDI TOF".

Un appareil de type "MALDI-TOF" est un spectromètre de masse couplant une source d'ionisation laser assistée par une matrice "MALDI", de l'anglais *"Matrix-Assisted Laser Desorption*/*Ionisation"*) et un analyseur à temps de vol ("TOF", de l'anglais *"time-of-flight mass spectrometry"*)*.*

Selon une deuxième variante de réalisation, un ou plusieurs appareils 2 peuvent être montés en série derrière un premier appareil 2, ce premier appareil étant alors préférentiellement mais non obligatoirement un appareil de chromatographie liquide et les autres appareils étant choisis parmi la liste précitée.

Les molécules séparées par cet appareil de chromatographie liquide sont alors analysées par le ou les appareils d'analyse monté(s) en série derrière.

Enfin, selon une autre variante de réalisation, le ou les appareils d'analyse 2 précités peuvent être remplacés par un unique collecteur de fractions, qui permet de collecter des échantillons du fluide issu du bioréacteur 1, accompagnés éventuellement d'un étalon interne, pour pouvoir les analyser avec un autre appareil, disposés par exemple dans un laboratoire distant.

De façon avantageuse, la boucle d'injection 15 est équipée d'une vanne à trois voies 5. Celle-ci comprend une entrée de raccordement 51 reliée à la boucle 15 et deux sorties, l'une 52 connectée à l'appareil 2 et l'autre 56 connectée à un réservoir de vidange 72.

Dans la première position de la vanne 5, représentée sur la figure 1, l'entrée 51 est raccordée à la sortie 52, de sorte que le contenu de la boucle d'injection 15 est envoyé à l'appareil 2. Dans la seconde position, représentée sur la figure 2, ce contenu est envoyé vers la vidange 72.

Cette vanne 5 à trois voies pourrait être remplacée par une vanne équipée d'un plus grand nombre de voies, par exemple six, comme représenté sur les figures. Dans ce cas les orifices de raccordement 53, 54 et 55 sont obturés par des bouchons.

L'installation de couplage entre le bioréacteur 1 et l'appareil ou les appareils 2, va maintenir être décrite plus en détail.

De manière générale, cette installation comprend une boucle de prélèvement 13, une boucle de nettoyage 14 et deux vannes à six voies, respectivement une première vanne 3 et une deuxième vanne 4.

Une vanne à six voies comprend un corps muni de six orifices de raccordement, ainsi qu'un élément d'obturation (clapet), mobile entre deux positions. Cet élément mobile permet de raccorder deux à deux les orifices de raccordement. En fonction de la position de l'élément mobile, des orifices de raccordement différents sont raccordés les uns aux autres.

La boucle de prélèvement 13 est une boucle qui forme une dérivation à partir de la boucle de perfusion fermée 11.

Comme on peut le voir sur les figures, les premier et deuxième orifices de raccordement 31, 32 de la vanne 3 sont reliés aux deux extrémités de la boucle de perfusion 11. Par ailleurs, les troisième et sixième orifices de raccordement 33, 36 de cette même vanne sont reliés aux deux extrémités de la boucle de prélèvement 13.

Dans la première position de la vanne 3 représentée sur la figure 1, les boucles 11 et 13 ne sont pas connectées et la perfusion du bioréacteur 1 est maintenue.

Dans la deuxième position de la vanne 3 représentée sur la figure 2, les premier et sixième orifices de raccordement 31, 36 sont raccordés entre eux tout comme le sont les deuxième et troisième orifices 32, 33, de sorte que la boucle de prélèvement 13 est en connexion de fluide avec la boucle 11 et constitue un prolongement de celle-ci. Ainsi, pendant la phase de prélèvement de l'échantillon, la perfusion du bioréacteur 1 est poursuivie.

La boucle 13 de prélèvement est une boucle à volume constant de fluide. Ce volume, qui correspond à celui de l'échantillon prélevé, est déterminé par la longueur et le diamètre du tuyau utilisé pour former cette boucle 13.

Selon la variante de réalisation de la figure 2, cette boucle de dérivation 13 peut également comprendre une colonne de piégeage 8, qui permet de retenir et donc de concentrer certaines molécules ou métabolites, tandis que le reste du fluide retourne irriguer le bioréacteur 1.

La boucle de nettoyage 14 d'un produit de nettoyage relie un dispositif 6 d'injection d'un produit de nettoyage à un réservoir de vidange 71, via les vannes 3 et 4. Ce produit de nettoyage est de préférence de l'air sous pression ou de l'air additionné d'un produit tel que de l'acétone par exemple.

Plus précisément, le dispositif 6 est raccordé au deuxième orifice de raccordement 42 de la vanne 4, le troisième orifice 43 est connecté au quatrième orifice 34 de la vanne 3, le cinquième orifice 35 de la vanne 3 est relié au sixième orifice 46 de la vanne 4 et enfin le premier orifice 41 est relié au réservoir de vidange 71, apte à recueillir les éventuels résidus de produits chassés par l'air et ou l'acétone.

Dans la première position de la vanne 4, représentée sur la figure 2, les orifices de raccordement sont raccordés par paires, comme suit : 41 et 42, 43 et 44, 45 et 46. Le dispositif 6 est relié directement à la vidange 71 et la boucle de prélèvement 13 n'est pas nettoyée.

Au contraire, dans la seconde position de la vanne 4, représentée sur la figure 1, les orifices 41 et 46 d'une part et 42 et 43 d'autre part sont connectés et la boucle 13 est nettoyée et/ou séchée, pour autant que la vanne 3 soit dans la première position.

L'ensemble des vannes 3, 4 et 5 sont des électrovannes, pilotées à distance par une unité de commande 9, qui peut éventuellement être intégrée dans l'un des appareils d'analyse physico-chimique 2.

Les différentes étapes du procédé d'analyse physico-chimique ou de collecte d'échantillons susceptibles d'être mises en place à l'aide de cette installation vont maintenant être décrites plus en détail.

### Etape 1 :

La première vanne 3 est placée dans sa première position (figure 1), la deuxième vanne 4 dans sa seconde position (figure 1) et la vanne 5 dans sa seconde position (figure 2).

La culture est maintenue en mode dynamique dans le bioréacteur 1 puisque le fluide biologique circule dans la boucle 11 grâce à la pompe 12 et retourne dans le bioréacteur. La boucle de prélèvement 13 et la colonne de piégeage 8, si celle-ci est présente, sont nettoyées et/ou séchées. Enfin, l'éluant est envoyé par l'injecteur 21 en direction du réservoir de vidange 72.

### Etape 2 :

La vanne 3 est basculée dans sa deuxième position (figure 2), la vanne 4 dans sa deuxième position (figure 2) et la vanne 5 dans sa première position (figure 1).

Un étalon interne est injecté depuis l'injecteur 21 via la boucle 14, les orifices de raccordement 35 et 34 de la vanne 3 puis les orifices 43 et 44 de la vanne 4 jusque dans l'appareil. Cet étalon interne permet de pallier l'encrassage de cet appareil. En effet, les fluides biologiques (milieux de culture, sérum etc..) ont tendance à encrasser ces appareils. Le nettoyage des appareils ne peut pas être envisagé pendant le cycle d'analyse. Cet étalon interne permet d'établir une référence qui sert à cibler et étalonner les courbes obtenues à l'aide des appareils et d'ainsi de limiter la perte de signal dans le temps, (notamment pour les durées d'analyse supérieures à 24 heures).

C'est l'injection de cet étalon interne qui déclenche le basculement de la vanne 3 dans la position précitée. De ce fait, la boucle de prélèvement 13 est raccordée à la boucle 11, ce qui permet de recueillir un échantillon dans la boucle d'injection 13, voire de piéger cet échantillon dans la colonne de piégeage 8. Dans ce dernier cas, la circulation au travers des boucles 11 et 13 est maintenue jusqu'à ce qu'une concentration suffisante de métabolites soit obtenue dans la colonne 8.

### Etape 3:

Ensuite, et comme représenté sur la figure 3, les vannes 3, 4 et 5 sont placées dans leur première position.

Dans ce cas, l'éluant injecté par l'injecteur 21 traverse la boucle 14, puis la boucle de prélèvement 13 et entraîne de ce fait le volume d'échantillon qui se trouvait dans cette boucle 13, jusque dans le ou les appareils.

Le cycle précité peut ensuite être répété, ce qui permet de nettoyer de nouveau la boucle de prélèvement 13, avant une nouvelle analyse ou un nouveau prélèvement.

L'installation conforme à l'invention présente de nombreux avantages.

Elle permet de réaliser des analyses en continu ou quasiment en continu (à l'exception de la période de nettoyage) des fluides produits par le bioréacteur et ce, tout en maintenant celui-ci en mode de culture dynamique.

Elle permet de nettoyer la boucle de prélèvement 13 et ce, sans polluer la boucle de perfusion 11. De façon avantageuse, un piège à bulle peut être présent dans la boucle de perfusion 11 pour piéger l'air encore présent dans la boucle 13 et qui pourrait y être entrainé lors du basculement de la vanne 3 dans la deuxième position.

Elle permet, grâce à l'injection de l'étalon interne, de conserver un niveau de précision élevé du signal obtenu par les appareils d'analyse 2, et ce, même pour des durées d'analyses longues (d'au moins 24 heures).

Enfin, l'installation peut être facilement démontée et transportée à proximité des appareils d'analyse et différents bioréacteurs peuvent aisément être connectés à l'installation pour effectuer des analyses successives.

## Revendications

1. Installation de couplage d'un bioréacteur (1) de culture de bio-organismes, en mode dynamique, avec au moins un appareil (2) d'analyse physico-chimique d'un fluide issu de ce bioréacteur ou de collecte d'échantillons de ce fluide, ledit appareil (2) étant équipé d'une pompe (20) et d'un injecteur (21), le bioréacteur (1) étant équipé de moyens (10) d'alimentation en milieu de culture et en xénobiotique(s), **caractérisée en ce que** le bioréacteur (1) est raccordé à une boucle de perfusion fermée (11) pourvue d'une pompe (12), cette boucle de perfusion (11) étant munie d'une boucle de dérivation, dite "boucle de prélèvement" (13) et **en ce que** l'installation comprend :
- un dispositif d'injection d'un produit de nettoyage (6) dans ladite boucle de prélèvement (13),
- une première vanne (3) comprenant six voies,
- et une deuxième vanne (4) comprenant six voies,
la première vanne (3) étant connectée à la boucle de perfusion fermée (11), à la boucle de prélèvement (13) et à une boucle de nettoyage (14), la deuxième vanne (4) étant raccordée à la boucle de nettoyage (14) et à une boucle d'injection (15) qui relie l'injecteur (21) à au moins l'un des appareils d'analyse physico-chimique ou de collecte d'échantillons (2).

2. Installation selon la revendication 1, **caractérisée en ce qu'**elle comprend une troisième vanne (5) munie d'au moins une entrée (51) et de deux sorties (52, 56), cette troisième vanne (5) étant positionnée sur la boucle d'injection (15), de façon que son entrée (51) soit connectée à l'injecteur (21), que sa première sortie (52) soit connectée à au moins l'un appareils d'analyse ou de collecte (2) et que sa deuxième sortie (56) soit connectée à un réservoir de vidange (72), ladite troisième vanne (5) occupant soit une première position dans laquelle l'entrée (51) est connectée à sa première sortie (52), soit une seconde position dans laquelle l'entrée (51) est connectée à sa deuxième sortie (56).

3. Installation selon la revendication 1 ou 2, **caractérisée en ce que** :
- pour la première vanne (3), ses premier et deuxième orifices de raccordement (31, 32) sont connectés à la boucle de perfusion fermée (11) du bioréacteur (1), ses troisième et sixième orifices de raccordement (33, 36) sont connectés à la boucle de prélèvement (13), son quatrième orifice de raccordement (34) au troisième orifice de raccordement (43) de la deuxième vanne (4) et son cinquième orifice de raccordement (35) au sixième orifice de raccordement (46) de la deuxième vanne, ladite première vanne (3) occupant soit une première position, dans laquelle son premier orifice de raccordement (31) est connecté à son deuxième (32), son troisième (33) à son quatrième (34) et son cinquième (35) à son sixième (36), soit une seconde position, dans laquelle son premier orifice de raccordement (31) est connecté à son sixième (36), son deuxième (32) à son troisième (33) et son quatrième (34) à son cinquième (35),
- pour la deuxième vanne (4), son premier orifice de raccordement (41) est connecté à un réservoir de vidange (71), son deuxième orifice de raccordement (42) au dispositif d'injection d'un produit de nettoyage (6), son cinquième orifice de raccordement (45) à l'injecteur (21) et son quatrième orifice de raccordement (44) à au moins l'un des appareils d'analyse physico-chimique (2, 2'), cette deuxième vanne (4) occupant soit une première position dans laquelle son premier orifice de raccordement (41) est connecté à son deuxième (42), son troisième (43) à son quatrième (44) et son cinquième (45) à son sixième (46), soit une seconde position dans laquelle son premier orifice de raccordement (41) est relié à son sixième (46), son deuxième (42) à son troisième (43) et son quatrième (44) à son cinquième (45).

4. Installation selon les revendications 2 et 3, **caractérisée en ce que** le quatrième orifice de raccordement (44) de la deuxième vanne (4) est connecté à l'entrée (51) de la troisième vanne (5).

5. Installation selon l'une des revendications précédentes, **caractérisée en ce que** la boucle de prélèvement (13) inclut une colonne de piégeage (8).

6. Installation selon l'une des revendications précédentes, **caractérisée en ce qu'**un filtre (108) est présent sur la boucle de perfusion (11) entre le bioréacteur et la première vanne (3).

7. Installation selon l'une des revendications précédentes, **caractérisée en ce que** le bioréacteur (1) est placé à l'intérieur d'une enceinte (107) dans laquelle le taux de dioxyde de carbone et la température sont contrôlés.

8. Installation selon l'une des revendications précédentes, **caractérisée en ce que** l'appareil d'analyse physico-chimique (2) est un appareil de chromatographie liquide.

9. Installation selon la revendication 8, **caractérisée en ce que** l'appareil d'analyse physico-chimique (2) est un appareil de chromatographie liquide haute précision (HPLC).

10. Installation selon l'une des revendications 1 à 7, **caractérisé en ce que** l'appareil d'analyse physico-chimique (2') est choisi parmi un spectromètre de masse, notamment un spectromètre de masse doté d'une source d'ionisation pour liquide de type ESI, APCI ou APPI, un appareil de résonance magnétique nucléaire (RMN), un détecteur fluorométrique, un spectrophotomètre ou un appareil de couplage en ligne MALDI TOF.

11. Installation selon l'une des revendications 1 à 7, **caractérisée en ce que** l'appareil (2) est un collecteur de fractions permettant de collecter des échantillons du fluide issu du bioréacteur (1).

12. Installation selon l'une des revendications 1 à 11, **caractérisée en ce qu'**elle comprend un ou plusieurs appareil(s) d'analyse physico-chimique (2), monté(s) en série après un premier appareil d'analyse physico-chimique, ce premier appareil étant notamment un appareil de chromatographie liquide.

13. Installation selon l'une des revendications précédentes, **caractérisée en ce que** le bioréacteur (1) est une boite multi-réacteurs.

14. Installation selon l'une des revendications 1 à 12, **caractérisée en ce que** le bioréacteur (1) est une biopuce.

15. Installation selon la revendication 14, **caractérisée en ce que** la biopuce (1) est de type micro-fluidique.

16. Procédé d'analyse physico-chimique en ligne d'un fluide biologique issu d'un bioréacteur (1) ou de collecte d'échantillons de ce fluide, avec l'installation selon la revendication 3 ou selon l'une quelconque des revendications 4 à 14 combinée à la revendication 3, **caractérisé en ce qu'**il consiste à réaliser au moins une fois le cycle d'étapes suivant :
- a) mettre la première vanne (3) dans sa première position et la deuxième vanne (4) dans sa seconde position, de façon à nettoyer et sécher la boucle de prélèvement (13),
- b) mettre la première vanne (3) et la deuxième vanne (4) dans leur seconde position, de façon à recueillir un échantillon dans la boucle de prélèvement (13) et à injecter un étalon interne dans l'appareil (2) à partir de l'injecteur (21),
- c) mettre la première vanne (3) et la deuxième vanne (4) dans leur première position, de façon à injecter l'échantillon recueilli dans la boucle de prélèvement (13), dans l'appareil (2), à l'aide d'un éluant fourni par l'injecteur (21).

## Patentansprüche

1. Anlage zum Koppeln eines Bioreaktors (1) für die Kultur von Bioorganismen im dynamischen Modus mit mindestens einem Gerät (2) für die physikalisch-chemische Analyse eines Fluids aus diesem Bioreaktor oder für die Probensammlung dieses Fluids, wobei das Gerät (2) mit einer Pumpe (20) und einem Injektor (21) ausgestattet ist, wobei der Bioreaktor (1) mit Versorgungsmitteln (10) mit Kulturmilieu und Xenobiotikum/Xenobiotika ausgestattet ist, **dadurch gekennzeichnet, dass** der Bioreaktor (1) mit einer geschlossenen Perfusionsschleife (11) gekoppelt ist, die mit einer Pumpe (12) ausgestattet ist, wobei diese Perfusionsschleife (11) mit einer Abzweigschleife, als "Entnahmeschleife" (13) bezeichnet, ausgestattet ist, und dass die Anlage umfasst:
- eine Injektionsvorrichtung eines Reinigungsprodukts (6) in die Entnahmeschleife (13),
- ein erstes Ventil (3), umfassend sechs Wege,
- und ein zweites Ventil (4), umfassend sechs Wege,
wobei das erste Ventil (3) mit der geschlossenen Perfusionsschleife (11), der Entnahmeschleife (13) und einer Reinigungsschleife (14) verbunden ist, wobei das zweite Ventil (4) an die Reinigungsschleife (14) und an eine Injektionsschleife (15) gekoppelt ist, die den Injektor (21) mit mindestens einem der Geräte für die physikalisch-chemische Analyse oder die Probensammlung (2) verbindet.

2. Anlage nach Anspruch 1, **dadurch gekennzeichnet, dass** sie ein drittes Ventil (5) umfasst, das mit mindestens einem Eingang (51) und mit zwei Ausgängen (52, 56) ausgestattet ist, wobei dieses dritte Ventil (5) derart auf der Injektionsschleife (15) positioniert ist, dass sein Eingang (51) mit dem Injektor (21) verbunden ist, dass sein erster Ausgang (52) mit mindestens einem der Geräte (2) für Analyse oder Sammel verbunden ist und dass sein zweiter Ausgang (56) mit einem Ablassbehälter (72) verbunden ist, wobei das dritte Ventil (5) entweder eine erste Position einnimmt, in welcher der Eingang (51) mit seinem ersten Ausgang (52) verbunden ist, oder eine zweite Position, in welcher der Eingang (51) mit seinem zweiten Ausgang (56) verbunden ist.

3. Anlage nach Anspruch 1 oder 2, **dadurch gekennzeichnet, dass**:
- für das erste Ventil (3) seine erste und zweite Anschlussöffnung (31, 32) mit der geschlossenen Perfusionsschleife (11) des Bioreaktors (1) verbunden sind, seine dritte und sechste Anschlussöffnung (33, 36) mit der Entnahmeschleife (13) verbunden sind, seine vierte Anschlussöffnung (34) mit der dritten Anschlussöffnung (43) des zweiten Ventils (4) und seine fünfte Anschlussöffnung (35) mit der sechsten Anschlussöffnung (46) des zweiten Ventils verbunden sind, wobei das erste Ventil (3) entweder eine erste Position einnimmt, in welcher seine erste Anschlussöffnung (31) mit seiner zweiten (32), seine dritte (33) mit seiner vierten (34) und seine fünfte (35) mit seiner sechsten (36) verbunden ist, oder eine zweite Position, in welcher seine erste Anschlussöffnung (31) mit seiner sechsten (36), seine zweite (32) mit seiner dritten (33) und seine vierte (34) mit seiner fünften (35) verbunden ist,
- für das zweite Ventil (4) seine erste Anschlussöffnung (41) mit einem Ablassbehälter (71), seine zweite Anschlussöffnung (42) mit der Injektionsvorrichtung eines Reinigungsprodukts (6), seine fünfte Anschlussöffnung (45) mit dem Injektor (21) und seine vierte Anschlussöffnung (44) mit mindestens einem der Geräte für die physikalisch-chemische Analyse (2, 2') verbunden ist, wobei dieses zweite Ventil (4) entweder eine erste Position einnimmt, in welcher seine erste Anschlussöffnung (41) mit seiner zweiten (42), seine dritte (43) mit seiner vierten (44) und seine fünfte (45) mit seiner sechsten (46) verbunden ist, oder eine zweite Position, in welcher seine erste Anschlussöffnung (41) mit seiner sechsten (46), seine zweite (42) mit seiner dritten (43) und seine vierte (44) mit seiner fünften (45) verbunden ist.

4. Anlage nach den Ansprüchen 2 und 3, **dadurch gekennzeichnet, dass** die vierte Anschlussöffnung (44) des zweiten Ventils (4) mit dem Eingang (51) des dritten Ventils (5) verbunden ist.

5. Anlage nach einem der vorangehenden Ansprüche, **dadurch gekennzeichnet, dass** die Entnahmeschleife (13) eine Abfangsäule (8) einschließt.

6. Anlage nach einem der vorangehenden Ansprüche, **dadurch gekennzeichnet, dass** auf der Perfusionsschleife (11) zwischen dem Bioreaktor und dem ersten Ventil (3) ein Filter (108) vorhanden ist.

7. Anlage nach einem der vorangehenden Ansprüche, **dadurch gekennzeichnet, dass** der Bioreaktor (1) im Innern eines Behälters (107) platziert ist, in welchem der Kohlendioxidgehalt und die Temperatur überwacht werden.

8. Anlage nach einem der vorangehenden Ansprüche, **dadurch gekennzeichnet, dass** das Gerät für die physikalisch-chemische Analyse (2) ein Flüssigchromatographiegerät ist.

9. Anlage nach Anspruch 8, **dadurch gekennzeichnet, dass** das Gerät für die physikalisch-chemische Analyse (2) ein Hochleistungs-Flüssigchromatographiegerät (HPLC) ist.

10. Anlage nach einem der Ansprüche 1 bis 7, **dadurch gekennzeichnet, dass** das Gerät für die physikalisch-chemische Analyse (2') aus einem Massenspektrometer, insbesondere aus einem Massenspektrometer mit einer Ionisierungsquelle für Flüssigkeit vom Typ ESI, APCI oder APPI, einem Nuklearmagnetresonanzgerät (NRM), einen fluorometrischen Detektor, einem Spektrophotometer oder einem Online-MALDI TOF-Kopplungsgerät ausgewählt ist.

11. Anlage nach einem der Ansprüche 1 bis 7, **dadurch gekennzeichnet, dass** das Gerät (2) ein Fraktionskollektor ist, der erlaubt, Proben des Fluids aus dem Bioreaktor (1) zu sammeln.

12. Anlage nach einem der Ansprüche 1 bis 11, **dadurch gekennzeichnet, dass** sie ein oder mehrere Geräte für die physikalisch-chemische Analyse (2) umfasst, die nach einem ersten Gerät für die physikalisch-chemische Analyse in Reihe montiert sind, wobei dieses erste Gerät insbesondere ein Flüssigchromatographiegerät ist.

13. Anlage nach einem der vorangehenden Ansprüche, **dadurch gekennzeichnet, dass** der Bioreaktor (1) ein Mehrfachreaktorgehäuse ist.

14. Anlage nach einem der Ansprüche 1 bis 12, **dadurch gekennzeichnet, dass** der Bioreaktor (1) ein Biochip ist.

15. Anlage nach Anspruch 14, **dadurch gekennzeichnet, dass** der Biochip (1) vom Typ Mikrofluid ist.

16. Verfahren für die physikalisch-chemische Online-Analyse eines biologischen Fluids aus einem Bioreaktor (1) oder für die Probensammlung dieses Fluids mit der Anlage nach Anspruch 3 oder nach einem der Ansprüche 4 bis 14, kombiniert mit Anspruch 3, **dadurch gekennzeichnet, dass** es darin besteht, mindestens einmal den folgenden Schrittzyklus zu absolvieren:
- a) Versetzen des ersten Ventils (3) in seine erste Position und des zweiten Ventils (4) in seine zweite Position zum Reinigen und Trocknen der Entnahmeschleife (13),
- b) Versetzen des ersten Ventils (3) und des zweiten Ventils (4) in ihre zweite Position zum Entnehmen einer Probe aus der Entnahmeschleife (13) und Einleiten eines internen Standards in das Gerät (2) ab dem Injektor (21),
- c) Versetzen des ersten Ventils (3) und des zweiten Ventils (4) in ihre erste Position, um die aus der Entnahmeschleife (13) entnommene Probe, mit Hilfe von dem Injektor (21) bereitgestellten Eluat, in das Gerät (2) einzuleiten.

## Claims

1. A coupling facility (1) of a bioreactor for culturing bio-organisms, in dynamic mode, with at least one unit (2) for physicochemical analysis of fluid coming from this bioreactor or for collecting samples of this fluid, said unit (2) being equipped with a pump (20) and an injector (21), the bioreactor (1) being equipped with means (10) of supplying culture medium and xenobiotic(s), **characterized in that** the bioreactor (1) is connected to a closed perfusion loop (11) provided with a pump (12), this perfusion loop (11) being fitted with a derivation loop, so-called "sample loop" (13) and **in that** the facility comprises:
- an injection device of a cleaning product (6) into said sample loop (13),
- a first valve (3) comprising six ways,
- and a second valve (4) comprising six ways,
the first valve (3) being connected to the closed perfusion loop (11), to the sample loop (13) and to a cleaning loop (14), the second valve (4) being connected to the cleaning loop (14) and to an injection loop (15) which connects the injector (21) to at least one of the units for physicochemical analysis or collecting samples (2).

2. Installation according to Claim 1, **characterized in that** it comprises a third valve (5) fitted with at least one input (51) and two outputs (52, 56), this third valve (5) being positioned on the injection loop (15) such that its input (51) is connected to the injector (21), its first output (52) is connected to at least one of the units for analysis or collecting (2) and its second output (56) is connected to a drain tank (72), said third valve (5) occupying either a first position in which the input (51) is connected to its first output (52), or a second position in which the input (51) is connected to its second output (56).

3. The facility according to Claim 1 or 2, **characterized in that**:
- for the first valve (3), its first and second connecting orifices (31, 32) are connected to the closed perfusion loop (11) of the bioreactor (1), its third and sixth connecting orifices (33, 36) are connected to the sample loop (13), its fourth connecting orifice (34) to the third connecting orifice (43) of the second valve (4) and its fifth connecting orifice (35) to the sixth connecting orifice (46) of the second valve, said first valve (3) occupying either a first position, in which its first connecting orifice (31) is connected to its second (32), its third (33) to its fourth (34) and its fifth (35) to its sixth (36), or a second position, in which its first connecting orifice (31) is connected to its sixth (36), its second (32) to its third (33) and its fourth (34) to its fifth (35),
- for the second valve (4), its first connecting orifice (41) is connected to a drain tank (71), its second connecting orifice (42) to the injection device of a cleaning product (6), its fifth connecting orifice (45) to the injector (21) and its fourth connecting orifice (44) to at least one of the physicochemical analysis units (2, 2'), this second valve (4) occupying either a first position in which its first connecting orifice (41) is connected to its second (42), its third (43) to its fourth (44) and its fifth (45) to its sixth (46), or a second position in which its first connecting orifice (41) is connected to its sixth (46), its second (42) to its third (43) and its fourth (44) to its fifth (45).

4. The facility according to Claims 2 and 3, **characterized in that** the fourth connecting orifice (44) of the second valve (4) is connected to the input (51) of the third valve (5).

5. The facility according to one of the preceding claims, **characterized in that** the sample loop (13) includes a trapping column (8).

6. The facility according to one of the preceding claims, **characterized in that** a filter (108) is present on the perfusion loop (11) between the bioreactor and the first valve (3).

7. The facility according to one of the preceding claims, **characterized in that** the bioreactor (1) is placed inside an enclosure (107) in which the rate of carbon dioxide and the temperature are controlled.

8. The facility according to one of the preceding claims, **characterized in that** the physicochemical analysis unit (2) is a liquid chromatography unit.

9. The facility according to Claim 8, **characterized in that** the physicochemical analysis unit (2) is a high-performance liquid chromatography unit (HPLC).

10. The facility according to one of Claims 1 to 7, **characterized in that** the physicochemical analysis unit (2') is selected from a mass spectrometer, especially a mass spectrometer provided with a source of ionisation for liquid of type ESI, APCI or APPI, a nuclear magnetic resonance unit (NMR), a fluorometric detector, a spectrophotometer or an online coupling unit MALDI TOF.

11. The facility according to one of Claims 1 to 7, **characterized in that** the unit (2) is a collector of fractions for collecting samples of fluid coming from the bioreactor (1).

12. The facility according to one of Claims 1 to 11, **characterized in that** it comprises one or more physicochemical analysis units (2), mounted in series after a first physicochemical analysis unit, this first unit being especially a liquid chromatography unit.

13. The facility according to one of the preceding claims, **characterized in that** the bioreactor (1) is a multi-reactor box.

14. The facility according to one of Claims 1 to 12, **characterized in that** the bioreactor (1) is a biochip.

15. The facility according to Claim 14, **characterized in that** the biochip (1) is of micro-fluidic type.

16. A process for online physicochemical analysis of a biological fluid coming from a bioreactor (1) or for collecting samples of this fluid, with the facility according to Claim 3 or according to any one of Claims 4 to 14 combined with Claim 3, **characterized in that** it consists of performing at least once the following cycle of steps:
- a) putting the first valve (3) in its first position and the second valve (4) in its second position, so as to clean and dry the sample loop (13),
- b) putting the first valve (3) and the second valve (4) in their second position, so as to collect a sample in the sample loop (13) and inject an internal standard in the unit (2) from the injector (21),
- c) putting the first valve (3) and the second valve (4) in their first position, so as to inject the sample collected into the sample loop (13), in the unit (2), by means of an eluent provided by the injector (21).
